(19) Europäisches Patentamt
European Patent Office
Office européen des brevets

(11) Numéro de publication: **0 176 435**
**B1**

(12) # FASCICULE DE BREVET EUROPÉEN

(45) Date de publication du fascicule du brevet: **12.10.88**

(51) Int. Cl.⁴: **C 07 D 311/72**

(21) Numéro de dépôt: **85401817.3**

(22) Date de dépôt: **19.09.85**

(54) **Dérivés du tocophérol, leur préparation et leur emploi.**

(30) Priorité: **20.09.84 FR 8414426**
**15.03.85 FR 8503843**

(43) Date de publication de la demande:
**02.04.86 Bulletin 86/14**

(45) Mention de la délivrance du brevet:
**12.10.88 Bulletin 88/41**

(84) Etats contractants désignés:
**AT BE CH DE FR GB IT LI LU NL SE**

(56) Documents cité:
**INDUSTRIE CHIMIQUE BELGE, vol. 35, no. 1, janvier 1970, pages 13-25; B. STALLA-BOURDILLON: "Mises au point sur les synthèses de la vitamine E (alpha-tocophérol)"**

(73) Titulaire: **RHONE- POULENC SANTE, Les Miroirs 18 Avenue d'Alsace, F-92400 Courbevoie Cédex (FR)**

(72) Inventeur: **Chabardes, Pierre, 24 rue Jeanne d'Arc, F-69110 Sainte Foy Les Lyon (FR)**
Inventeur: **Mulhauser, Michel, Immmeuble "Frênes 4" Résidence Charrière Blanche, F-69130 Ecully (FR)**

(74) Mandataire: **Pilard, Jacques, RHONE- POULENC RECHERCHES Service Brevets Pharma 25, Quai Paul Doumer, F-92408 Courbevoie Cedex (FR)**

EP 0 176 435 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne de nouveaux dérivés du tocophérol de formule générale (I):

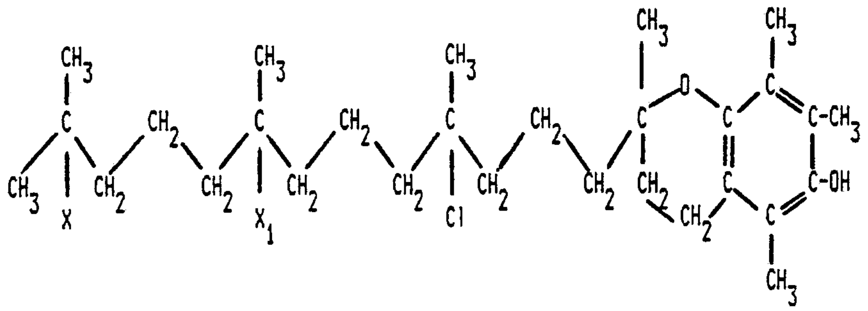

(I)

et leurs acétates, leur préparation et leur emploi, en particulier, dans la synthèse de la vitamine E.

Dans la formule générale (I), les symboles X et $X_1$, identiques ou différents, représentent un atome d'hydrogène ou de chlore.

Il est connu de préparer la vitamine E par condensation du phytol ou de ses dérivés sur la triméthylhydroquinone [B. Stalla - Bourdillon, Industrie Chimique Belge 35, (1), 13 - 25 (1970)]; le phytol ou ses dérivés étant généralement obtenus à partir de la pseudo-ionone par des méthodes de synthèse nécessitant la réalisation d'un grand nombre d'étapes.

Selon l'invention, les nouveaux produits de formule générale (I) peuvent être obtenus par condensation d'un dérivé chloré de l'hexadécène de formule générale (IIa) ou (IIb)

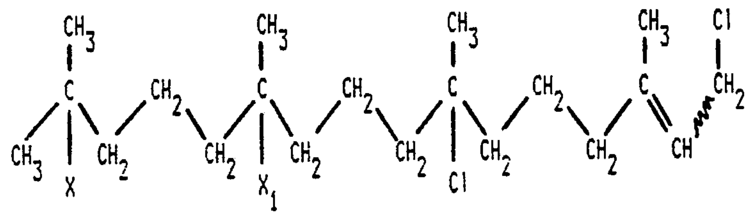

(IIa)

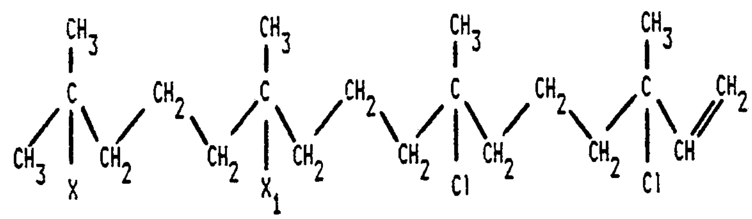

(IIb)

dans laquelle les symboles X et $X_1$, identiques ou différents, représentent un atome d'hydrogène ou de chlore, ou leurs mélanges, sur la triméthylhydroquinone.

Généralement la condensation s'effectue en présence de chlorure de zinc en opérant dans un solvant organique choisi parmi l'acide acétique et le dioxanne à une température comprise entre 0 et 50° C.

L'acétate d'un produit de formule générale (I) peut être obtenu par acétylation du produit de formule générale (I) au moyen d'anhydride acétique en présence de chlorure de zinc ou en présence d'un mélange de triéthylamine et de diméthylaminopyridine à une température voisine de 20° C.

Les produits de formule générale (IIa) et (IIb) peuvent être obtenus par hydrochloration d'un polyène de formule générale (III):

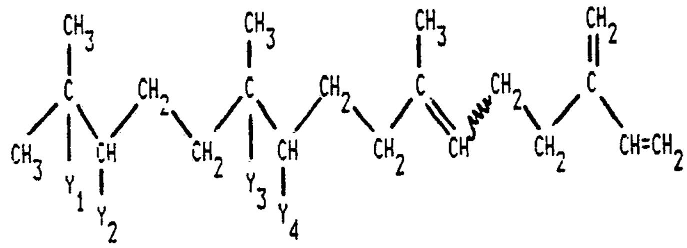

(III)

dans laquelle

$Y_1$ représente un atome d'hydrogène ou de chlore,

$Y_2$ représente un atome d'hydrogène ou bien $Y_1$ et $Y_2$ forment ensemble une liaison et

$Y_3$ et $Y_4$ représentent un atome d'hydrogène ou forment ensemble une liaison,

au moyen d'acide chlorhydrique anhydre en opérant en présence d'un halogénure cuivreux, tel que le chlorure ou l'iodure cuivreux, associé à un sel d'ammonium quaternaire choisi parmi les halogénures de tétraalcoylammonium et les halohydrates de trialcoylamine, ou à un sel de phosphonium choisi parmi les halogénures de tétraalcoylphosphonium, dans un solvant organique inerte choisi parmi les hydrocarbures aliphatiques halogénés (chlorure de méthylène), les acides carboxyliques (acide acétique), les anhydrides d'acide carboxylique (anhydride acétique), les hydrocarbures aliphatiques (hexane), cycloaliphatiques (cyclohexane) ou aromatiques (benzène) à une température inférieure à 20°C et, de préférence, inférieure à 0°C, étant entendu que:

- lorsque les symboles Y représentent chacun un atome d'hydrogène, on utilise au moins deux moles d'acide chlorhydrique anhydre par mole de produit de formule générale (III), et
- lorsque les symboles Y forment ensemble une liaison, on utilise au moins trois moles d'acide chlorhydrique anhydre par mole de produit de formule générale (III).

Les produits de formule générale (III) dans laquelle $Y_3$ et $Y_4$ représentent chacun un atome d'hydrogène et $Y_1$ et $Y_2$ représentent chacun un atome d'hydrogène ou forment ensemble une liaison peuvent être préparés dans les conditions décrites dans le brevet américain US-4 292 459.

Le produit de formule générale (III) dans laquelle $Y_1$ représente un atome de chlore, $Y_2$ représente un atome d'hydrogène et $Y_3$ et $Y_4$ forment ensemble une liaison peut être obtenu à partir du myrcène en conduisant le dérivé magnésien du dichloro-1,7 diméthyl-3,7 octène sur le chloro-3 myrcène.

Le dichloro-1,7 diméthyl-3,7 octène peut être obtenu en faisant réagir sur le myrcène au moins deux moles d'acide chlorhydrique anhydre par mole de myrcène en présence d'un catalyseur constitué d'un halogénure cuivreux, tel que le chlorure ou l'iodure cuivreux, associé à un sel d'ammonium quaternaire, choisi parmi les halogénures de tétraalcoylammonium et les halohydrates de trialcoylamines, ou à un sel de phosphonium choisi parmi les halogénures de tétraalcoylphosphonium, dans un solvant organique inerte choisi parmi les hydrocarbures aliphatiques halogénés (chlorure de méthylène), les acides carboxyliques (acide acétique), les anhydrides d'acides carboxyliques (anhydride acétique), les hydrocarbures aliphatiques (hexane), cycloaliphaliques (cyclohexane) ou aromatiques (benzène) à une température inférieure à 20°C et de préférence inférieure à 0°C.

Le magnésien du dichloro-1,7 diméthyl-3,7 octène est obtenu dans les conditions habituelles par action du dichloro-1,7 diméthyl-3,7 octène sur le magnésium dans un solvant organique choisi parmi les éthers (éther éthylique, tétrahydrofuranne) à une température inférieure à 0°C.

La condensation du magnésien du dichloro-1,7 diméthyl-3,7 octène sur le chloro-3 myrcène s'effectue généralement à une température inférieure à 0°C dans un solvant organique choisi parmi les éthers (éther éthylique, tétrahydrofuranne) en présence d'un halogénure cuivreux tel que l'iodure cuivreux.

Le produit de formule générale (III) dans laquelle $Y_1$ et $Y_2$ et $Y_3$ et $Y_4$ forment respectivement une liaison, c'est-à-dire le β-springène, peut être obtenu par action du dérivé magnésien des chlorures de géranyle et de néryle sur le chloro-3 myrcène.

Le mélange des chlorures de géranyle et de néryle peut être obtenu par hydrochloration du myrcène en présence d'une mole d'acide chlorhydrique anhydre par mole de myrcène dans les conditions décrites précédemment pour l'obtention du dichloro-1,7 diméthyl-3,7 octène.

Le magnésien du mélange des chlorures de géranyle et de néryle peut être obtenu dans les conditions décrites précédemment pour l'obtention du magnésien du dichloro-1,7 diméthyl-3,7 octène.

Les produits de formule générale (I) et leurs acétates obtenus selon le procédé de la présente invention sont particulièrement utiles dans la synthèse de la vitamine E.

Par exemple, l'hydrogénation d'un produit de formule générale (I) ou de son acétate, effectuée au moyen d'hydrogène en présence d'un catalyseur tel que la palladium sur charbon en opérant dans un solvant organique tel que l'acide acétique ou l'éthanol, à une température comprise entre 50 et 100°C, éventuellement sous pression, conduit au tocophérol ou à l'acétate de tocophérol.

Les exemples suivants, donnés à titre non limitatif, montrent comment l'invention peut être mise en pratique.

**Exemple 1**

Dans un réacteur de 250 $cm^3$, on introduit 8,4 g de triméthylhydroquinone, 22 g de tétrachloro-1,7,11,15 tétraméthyl-3,7,11,15 hexadécène-2 et 30 $cm^3$ d'acide acétique. On ajoute ensuite en 10 minutes une solution de 1,5 g de chlorure de zinc dans 15 $cm^3$ d'acide acétique anhydre. La température monte de 25 à 30°C. Le mélange réactionnel est agité pendant 2 heures à 30°C puis il est versé dans un mélange de 100 $cm^3$ d'hexane et de 100 $cm^3$ d'eau. La phase organique, séparée par décantation, est lavée par 100 $cm^3$ d'un mélange méthanol-eau (50 - 50 en volumes). Il se forme dans la phase hexanique un précipité blanc qui est séparé par filtration et lavé par 50 $cm^3$ d'un mélange méthanol-eau (50 - 50 en volumes). Après séchage sous pression réduite, on obtient 14,3 g de tétraméthyl-2,5,7,8 (trichloro-4',8',12' triméthyl-4',8',12' tridécyl)-2 chromanol-6 sous forme de cristaux blancs fondant à 102 - 104°C. Le rendement est de 62 %.

La structure du produit obtenu est confirmée par le spectre de masse et les spectres de résonance magnétique nucléaire du proton et du $^{13}C$.

Le tétrachloro-1,7,11,15 tétraméthyl-3,7,11,15 hexadécène-2 peut être préparé selon l'un des procédés suivants:

a) Dans un ballon tricol de 500 $cm^3$ muni d'une agitation magnétique, d'un thermomètre et d'un tube plongeant, on introduit, sous atmosphère d'argon, 3,4 g de chlorhydrate de triéthylamine, 2,5 g de chlorure cuivreux et 270 $cm^3$ de chlorure de méthylène. On refroidit à -10°C et, à la solution homogène jaune ainsi obtenue, on ajoute 136 g de myrcène (1 mole) dont la pureté est supérieure à 95 % puis, en 6 heures, 80 g d'acide chlorhydrique anhydre. La solution ainsi obtenue est maintenue à -25°C pendant 18 heures.

Le mélange réactionnel est versé dans un mélange de 400 $cm^3$ d'une solution aqueuse de chlorure d'ammonium à 10 % et de 300 $cm^3$ de pentane. Après décantation, la phase organique est lavée par 3 fois 200 $cm^3$ d'eau puis est séchée sur carbonate de potassium. Après filtration et évaporation du solvant, on obtient 237,8 g d'une huile jaune pâle contenant essentiellement le dichloro-1,7 diméthyl-3,7 octène-2 sous forme d'un mélange des isomères E et Z.

Dans un réacteur de 250 $cm^3$, on introduit 12,15 g de magnésium, 30 $cm^3$ de tétrahydrofuranne et un cristal d'iode. On refroidit à -20°C puis on ajoute en 5 heures 30 minutes une solution de 20,9 g de dichloro-1,7 diméthyl-3,7 octène-2 obtenu précédemment dans 85 $cm^3$ de tétrahydrofuranne. On poursuit l'agitation pendant 18 heures à -20°C. L'excès de magnésium est éliminé par filtration puis la solution obtenue est introduite, à l'abri de l'air et de l'humidité, dans une ampoule de coulée.

Dans un réacteur de 250 $cm^3$, on introduit 0,5 g d'iodure de cuivre et 5 $cm^3$ de tétrahydrofuranne puis on ajoute 1,5 $cm^3$ de solution magnésienne. On ajoute ensuite rapidement 19,5 g de chloro-3 myrcène, dont la pureté est supérieure à 87 %, dans 10 $cm^3$ de tétrahydrofuranne. On refroidit à -20°C puis on ajoute en 3 heures la solution magnésienne restante. On laisse la température remonter en 1 heure au voisinage de 20°C. On ajoute au mélange réactionnel de l'eau (5 $cm^3$) et du pentane (100 $cm^3$). La phase organique, séparée par décantation, est séchée sur sulfate de magnésium. Après filtration et évaporation du solvant, on obtient 29,7 g d'une huile.

D'après le dosage par chromatographie en phase vapeur avec étalon interne, le taux de transformation du chloro-3 myrcène est de 69 %.

L'huile obtenue est chauffée à 100 - 105°C sous pression réduite (0,5 - 1 mm de mercure ; 0,067 - 0,13 kPa) afin d'éliminer les produits en $C_{10}$ n'ayant pas réagi.

Le résidu obtenu (20 g) contient 85 % de chloro-15 méthylène-3 triméthyl-7,11,15 hexadécatriène-1,6,10.

Le rendement est de 82 % par rapport au chloro-3 myrcène consomme.

La structure du produit obtenu est confirmée par le spectre de masse et le spectre de résonance magnétique nucléaire du proton.

Dans un réacteur de 250 $cm^3$, on introduit, sous atmosphère d'argon, 0,48 g de chlorhydrate de triéthylamine, 15 $cm^3$ de chlorure de méthylène, 10 $cm^3$ d'acide acétique et 90 mg de chlorure cuivreux. On agite le mélange réactionnel jusqu'à l'obtention d'une solution homogène. On refroidit à -10°C puis on ajoute 10 g de chloro-15 méthylène-3 triméthyl-7,11,15 hexadécatriène-1,6,10 et, en 1 heure, 3,9 g d'acide chlorhydrique gazeux sec. Le mélange réactionnel est versé dans 100 $cm^3$ d'une solution aqueuse de chlorure d'ammonium à 100 g/litre. La phase organique est séparée par décantation puis on extrait la phase aqueuse par 2 fois 100 $cm^3$ de chlorure de méthylène. Les phases organiques réunies sont lavées par 100 $cm^3$ d'eau puis séchées sur carbonate de potassium. Après filtration et évaporation du solvant, on obtient 13,1 g de tétrachloro-1,7,11,15 tétraméthyl-3,7,11,15 hexadécène-2 avec un rendement de 96,5 %.

La structure du produit obtenu est confirmée par le spectre de masse.

b) Dans un réacteur de 250 $cm^3$, on introduit, sous atmosphère d'argon, 0,48 g de chlorhydrate de triéthylamine, 15 $cm^3$ de chlorure de méthylène, 10 $cm^3$ d'acide acétique et 90 mg de chlorure cuivreux. On agite le mélange réactionnel jusqu'à l'obtention d'une solution homogène. On refroidit à -10°C puis on ajoute 10 g de β-springène et, en 1 heure, 5,2 g d'acide chlorhydrique gazeux sec. Après traitement du mélange réactionnel dans les conditions décrites précédemment, on obtient 14,2 g de tétrachloro-1,7,11,15 tétraméthyl-3,7,11,15 hexadécène-2 avec un rendement de 94 %.

La structure du produit obtenu est confirmée après son hydrogénation en phytane.

**Exemple 2**

Dans un réacteur de 250 $cm^3$, on introduit 0,22 g de chlorure de zinc fondu, 2,47 g de triméthylhydroquinone et 10 $cm^3$ de dioxanne anhydre. On chauffe à 40 - 45°C puis on ajoute en 20 minutes une solution de 6,8 g de tétrachloro-1,7,11,15 tétraméthyl-3,7,11,15 hexadécène-2 dans 7 $cm^3$ de dioxanne. On poursuit l'agitation pendant 1 heure 30 minutes. Le mélange réactionnel est versé dans 50 $cm^3$ d'une solution aqueuse de chlorure d'ammonium à 100 g/litre. On extrait avec 2 fois 50 $cm^3$ d'acétate d'éthyle puis sèche les phases organiques sur sulfate de magnésium. Après filtration et évaporation du solvant on obtient le tétraméthyl-2,5,7,8 (trichloro-4',8',12' triméthyl-4',8',12' tridécyl)-2 chromanol-6 avec un rendement de 43,5 %.

**Exemple 3**

Dans un ballon tricol, on introduit, sous atmosphère d'argon, 2,1 g du produit obtenu à l'exemple 1, 150 mg de diméthylaminopyridine et 10 $cm^3$ de triéthylamine puis on ajoute rapidement 6 $cm^3$ d'anhydrique acétique sous agitation à une température de 25°C. Après une heure d'agitation, on ajoute 20 $cm^3$ d'eau puis neutralise le mélange réactionnel par addition progressive de carbonate de sodium jusqu'à cessation du dégagement de gaz carbonique. Le mélange réactionnel est extrait par 2 fois 50 $cm^3$ d'acétate d'éthyle. La phase organique est lavée par 3 fois 50 $cm^3$ d'une solution aqueuse d'acide chlorhydrique 0,1N. Les phases organiques sont séchées sur sulfate de magnésium. Après filtration et évaporation du solvant, le résidu obtenu est repris par l'hexane. Le précipité qui se forme est séparé par filtration. On obtient ainsi, avec un rendement de 93 %, l'acétate de tétraméthyl-2,5,7,8 (trichloro-4',8',12' triméthyl-4',8',12' tridécyl)-2 chromanol-6 fondant à 95-105°C.

La structure du produit obtenu est confirmée par le spectre de masse et les spectres de résonance magnétique nucléaire du proton et du $^{13}C$.

**Exemple 4**

Dans un réacteur, on introduit, sous atmosphère d'argon, 5 g du produit obtenu à l'exemple 1, 20 $cm^3$ d'acide acétique et 320 mg de chlorure de zinc anhydre. On ajoute 5 $cm^3$ d'une solution d'acide chlorhydrique dans l'acide acétique à 1,9 mole d'acide chlorhydrique par litre. On ajoute ensuite en 15 minutes, 2,7 $cm^3$ d'anhydride acétique. La température monte de 20 à 30°C. Après 2 heures d'agitation, on ajoute 10 $cm^3$ d'eau, 800 mg d'acétate de sodium et 100 $cm^3$ d'acétate d'éthyle. Après évaporation des solvants, le résidu est repris par du chlorure de méthylène. Après filtration sur gel de silice, on obtient 4,99 g d'acétate de tétraméthyl-2,5,7,8 (trichloro-4',8',12' triméthyl-4',8',12' tridécyl)-2 chromanol-6.

Le taux de transformation du tétraméthyl-2,5,7,8 (trichloro-4',8',12' triméthyl-4',8',12' tridécyl)-2 chromanol-6 est de 100 %. Le rendement est de 92,5 %.

**Exemple 5**

Dans un réacteur, on introduit, sous atmosphère d'argon, 186 mg de chlorure de zinc et 3 $cm^3$ d'acide acétique. On ajoute alors 1,85 g de triméthylhydroquinone, 1,5 $cm^3$ d'acide acétique et 4,5 $cm^3$ de chlorure de méthylène. On ajoute ensuite en 15 minutes et à 23°C, 5,1 g de tétrachloro-1,7,11,15 tétraméthyl-3,7,11,15 hexadécène-2 en solution dans 4 $cm^3$ d'acide acétique et 4 $cm^3$ de chlorure de méthylène. Après 2 heures d'agitation à une température comprise entre 22 et 25°C on ajoute 3,5 $cm^3$ d'anhydride acétique. La température monte à 32°C. Après 15 heures à une température voisine de 25°C, on ajoute 100 $cm^3$ d'eau puis du bicarbonate de sodium jusqu'à neutralité. On extrait par 2 fois 50 $cm^3$ d'acétate d'éthyle. Les phases organiques sont séchées sur carbonate de potassium. Après filtration et évaporation du solvant, on obtient 5,82 g d'une huile contenant 64 % d'acétate de tétraméthyl-2,5,7,8 (trichloro-4',8',12' triméthyl-4',8',12' tridécyl)-2 chromanol-6.

Le rendement est de 53 %.

**Exemple 6**

Dans un ballon tricol muni d'une agitation magnétique, d'un thermomètre et d'un réfrigérant surmonté d'une tête à hydrogéner, on introduit 1 g du produit obtenu à l'exemple 3, 20 $cm^3$ d'acide acétique et 0,1 g de palladium sur charbon à 10 % de palladium. Le mélange réactionnel est chauffé à 80°C sous pression atmosphérique d'hydrogène. La quantité théorique d'hydrogène est absorbée en 2 heures. Après refroidissement le catalyseur est séparé par filtration. Après évaporation du solvant on obtient 0,9 g d'une huile

jaune très pâle contenant 89,5 % en poids d'acétate de tocophérol.

**Exemple 7**

Dans un autoclave, on introduit 2,04 g du produit obtenu à l'exemple 1, 44 mg de palladium sur charbon à 10 % de palladium et 25 $cm^3$ d'éthanol. On établit une pression d'hydrogène de 50 bars puis chauffe à 80°C pendant 5 heures tout en agitant. Après refroidissement, séparation du catalyseur par filtration et évaporation du solvant, on obtient le tocophérol avec un rendement de 96 %.

**Exemple 8**

Dans un tricol de 250 $cm^3$, on introduit, sous atmosphère d'argon, 990 mg de chlorure de zinc anhydre (0,007 mole) que l'on dissout dans 20 $cm^3$ d'acide acétique. On ajoute ensuite 4,4 g de triméthylhydroquinone (0,0289 mole). On verse sur ce mélange hétérogène, en 40 minutes à une temperature comprise entre 20 et 26°C, 10 g d'un mélange de dichloro-1,7 tétraméthyl-3,7,11,15 hexadecène-2 et de dichloro-3,7 tétraméthyl-3,7,11,15 hexadécène-1 en solution dans 20 $cm^3$ d'acide acétique. Le mélange devient homogène et est de couleur rouge brun. Après 1 heure d'agitation, on ajoute 10 $cm^3$ d'anhydride acétique puis on maintient l'agitation pendant encore 2 heures. Après hydrolyse à l'eau, extraction à l'éther et séchage sur sulfate de magnésium, le solvant est évaporé sous pression réduite. On obtient ainsi 16,2 g d'une huile jaune dont l'analyse par spectrométrie de masse, résonance magnétique nucléaire du proton et du $^{13}C$ montre qu'elle est constituée essentiellement d'acétate de tétraméthyl-2,5,7,8 (chloro-4' triméthyl-4',8',12' tridécyl)-2 chromanol-6.

Le taux de transformation (déterminé par dosage du diacétate de triméthylhydroquinone récupéré) est de 80,4 %.

Le mélange de dichloro-1,7 tétraméthyl-3,7,11,15 hexadécène-2 et de dichloro-3,7 tétraméthyl-3,7,11,15 hexadécène-1 peut être préparé de la manière suivante:

Dans un tricol de 250 $cm^3$, on introduit, sous atmosphère d'argon, 360,5 mg de chlorhydrate de triéthylamine ($0,26 \times 10^{-2}$ mole), 126 mg de chlorure cuivreux ($0,13 \times 10^{-2}$ mole), 9 $cm^3$ d'acide acétique et 9 $cm^3$ de chlorure de méthylène. On agite jusqu'à l'obtention d'une solution homogène jaune. On refroidit à 0°C puis on ajoute rapidement 13,96 g de méthylène-3 triméthyl-7,11,15 hexadécadiène-1,6 dont la pureté est de 95 %. On refroidit la solution à une température voisine de -5°C puis on fait passer un courant d'acide chlorhydrique gazeux anhydre pendant 1 heure 20 minutes de façon à introduire 5 g (0,137 mole) d'acide chlorhydrique. Après 30 minutes d'agitation à une température voisine de -5°C, on verse le mélange réactionnel dans 20 $cm^3$ de pentane et 20 $cm^3$ d'une solution aqueuse de chlorure d'ammonium à 10 % en poids à une température voisine de 20°C. La phase organique est séparée par décantation puis séchée sur sulfate de sodium. Après filtration et évaporation du solvant, on obtient 17,31 g d'un produit brut dont l'analyse par spectrographie de masse et par résonance magnétique nucléaire du proton révèle la présence de 90 % d'un mélange de dichloro-1,7 tétraméthyl-3,7,11,15 hexadécène-2 et de dichloro-3,7 tétraméthyl-3,7,11,15 hexadécène-1.

Pour vérifier la linéarité du squelette du produit obtenu on traite 1,7 g du produit obtenu précédemment en solution dans 20 $cm^3$ d'éthanol, à 80°C sous une pression de 20 bars d'hydrogène en présence de 170 mg de palladium sur noir à 10 %. Après filtration du catalyseur et évaporation du solvant, le dosage par chromatographie en phase vapeur avec étalon interne montre que le rendement en phytane est de 83,7 % par rapport au triène mis en oeuvre.

La sélectivité en phytane par rapport aux autres isomères est de 98 %.

**Exemple 9**

On opère comme dans l'exemple 8 mais à partir des produits suivants:

| | |
|---|---|
| - mélange de trichloro-1,7,15 tétraméthyl-3,7,11,15 hexadécène-2 et de trichloro-3,7,15 tétraméthyl-3,7,11,15 hexadécène-1 | 10 g |
| - triméthylhydroquinone | 4 g |
| - chlorure de zinc | 914 mg |
| - acide acétique | 43 $cm^3$ |
| - anhydride acétique | 10 $cm^3$ |

Après traitement du mélange réactionnel, on obtient 16,63 g d'une huile orangée.

Le taux de transformation de la triméthylhydroquinone est de 81,3 % (détermination par dosage du diacétate de triméthylhydroquinone).

La structure de l'acétate de tétraméthyl-2,5,7,8 (dichloro-4',12' triméthyl-4',8',12' tridécyl)-2 chromanol-6 est

confirmée par le spectre de masse et les spectres de résonance magnétique nucléaire du proton et du $^{13}C$ à partir d'une fraction purifiée de l'huile obtenue.

Le mélange de trichloro-1,7,15 tétraméthyl-3,7,11,15 hexadécène-2 et de trichloro-3,7,15 tétraméthyl-3,7,11,15 hexadécène-1 peut être préparé de la manière suivante:

On opère comme dans l'exemple 1 mais à partir des produits suivants:

| | |
|---|---|
| - méthylène-2 triméthyl-7,11,15 hexadécatriène-1,6,14 | 14 g (5,1 x $10^{-2}$ mole) |
| - chlorhydrate de triéthylamine | 370 mg |
| - chlorure cuivreux | 130 mg |
| - acide acétique | 9 $cm^3$ |
| - chlorure de méthylène | 9 $cm^3$ |

On fait passer un courant d'acide chlorhydrique gazeux anhydre pendant 1 heure de façon à introduire 7,3 g d'acide chlorhydrique.

Après traitement du mélange réactionnel, on recueille 19,31 g d'une huile dont l'analyse par spectrométrie de masse et par résonance magnétique nucléaire du proton montre qu'elle est constituée essentiellement de trichloro-1,7,15 tétraméthyl-3,7,11,15 hexadécène-2 et de trichloro-3,7,15 tétraméthyl-3,7,11,15 hexadécène-1 et qu'elle ne contient pas de diènes conjugués.

L'hydrogénation du produit obtenu dans les conditions décrites dans l'exemple 1 montre que, d'après le dosage par chromatographie en phase vapeur avec étalon interne, le rendement en phytane est de 63 % par rapport au méthylène-2 triméthyl-7,11,15 hexadécatriène-1,6,14 mis en oeuvre.

**Exemple 10**

Dans un appareil à hydrogéner, on introduit 6,67 g du produit obtenu à l'exemple 8, 60 $cm^3$ d'acide acétique et 400 mg de palladium sur noir a 10 % en poids de palladium. On chauffe à 80°C pendant 2 heures 30 minutes sous une pression d'hydrogène de 1 bar. Après refroidissement, filtration du catalyseur et évaporation du solvant, on obtient 5,62 g d'une huile claire contenant 74,7 % d'acétate de tocophérol.

Le rendement en acétate de tocophérol est de 93 %, par rapport à la triméthylhydroquinone ayant réagi, et de 80 % par rapport au méthylène-3 triméthyl-7,11,15 hexadécadiène-1,6 ayant réagi.

Le taux de transformation du dichloro-1,7 tétraméthyl-3,7,11,15 hexadécène-2 et du dichloro-3,7 tétraméthyl-3,7,11,15 hexadécène-1 est de 97 %, la détermination étant effectuée par dosage du phytane récupéré.

**Exemple 11**

On dissout 2,9 g de l'huile obtenue à l'exemple 9 dans 30 $cm^3$ d'acide acétique contenant 220 mg de palladium sur charbon à 10 % en poids de palladium. On chauffe à 80°C pendant 4 heures 30 minutes sous une pression d'hydrogène de 1 bar. Après traitement du mélange réactionnel, on obtient 2,17 g d'une huile claire contenant 62 % d'acétate de tocophérol.

Le rendement en acétate de tocophérol est de 76,7 % par rapport à la triméthylhydroquinone ayant réagi et de 65 % par rapport au méthylène-2 triméthyl-7,11,15 hexadécatriène-1,6,14 ayant réagi.

Le taux de transformation du trichloro-1,7,15 tétraméthyl-3,7,11,15 hexadécène-2 et du trichloro-3,7,15 tétraméthyl-3,7,11,15 hexadécène-1 est de 97 %; la détermination étant effectuée par dosage du phytane récupéré.

**Revendications** pour les Etats contractants: BE, CH, DE, FR, GB, IT, LI, LU, NL, SE

1. Un dérivé du tocophérol de formule générale (I):

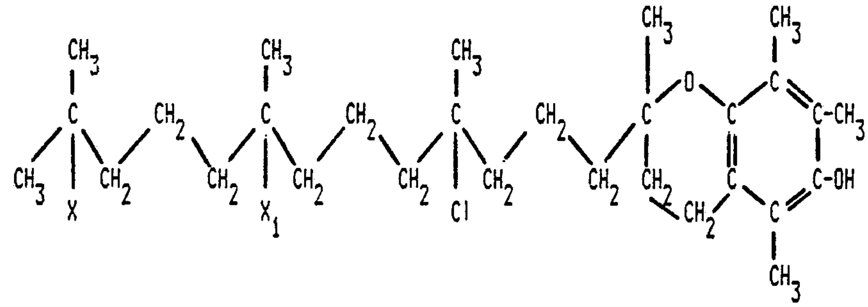

(I)

dans laquelle les symboles X et $X_1$ identiques ou différents, représentent un atome d'hydrogène ou de chlore, et son acétate.

2. Un procédé de préparation d'un produit selon la revendication 1 caractérisé en ce que l'on condense un produit de formule générale (IIa) ou (IIb):

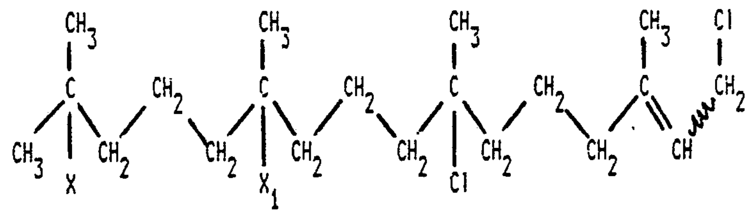

(IIa)

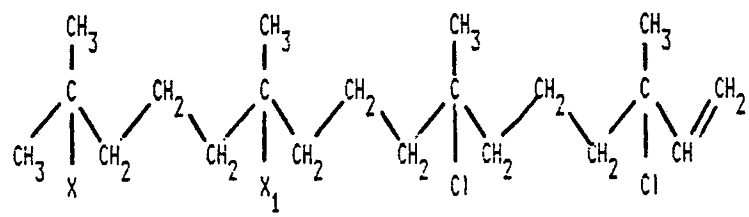

(IIb)

dans laquelle les symboles X et $X_1$, sont définis comme dans la revendication 1 ou ses mélanges, sur la triméthylhydroquinone en opérant dans un solvant organique choisi parmi l'acide acétique ou le dioxanne à une température comprise entre 0 et 50° C, puis, éventuellement, acétyle le produit de formule générale (I) ainsi obtenu au moyen d'anhydride acétique en présence de chlorure de zinc ou en présence d'un mélange de triéthylamine et de diméthylaminopyridine pour obtenir l'acétate du produit de formule (I).

**Revendication** pour l'Etat contractant: AT

Un procédé de préparation d'un dérivé du tocophérol de formule générale (I):

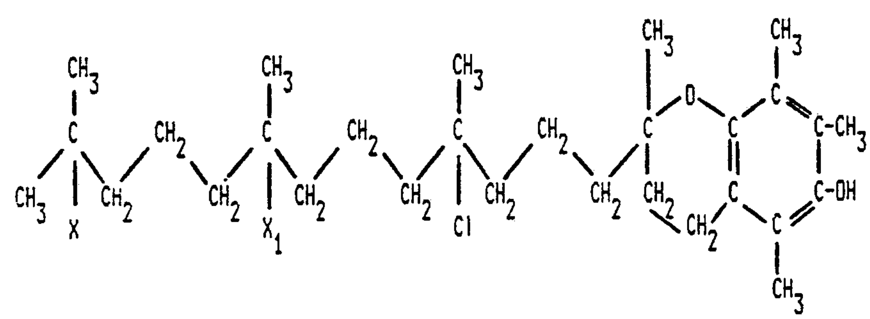

(I)

dans laquelle les symboles X et $X_1$ identiques ou différents, représentent un atome d'hydrogène ou de chlore, et de son acétate caractérisé en ce que l'on condense un produit de formule générale (IIa) ou (IIb):

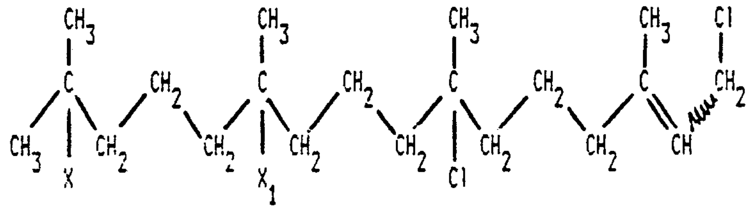

(IIa)

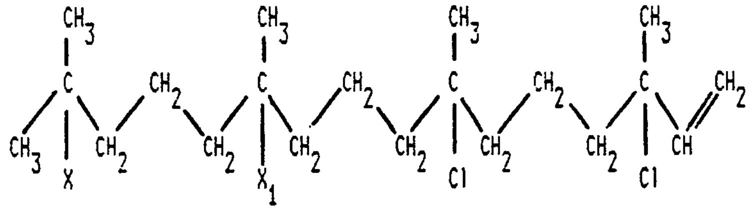

(IIb)

dans laquelle les symboles X et $X_1$, sont définis comme précédemment, ou ses mélanges, sur la triméthylhydroquinone en opérant dans un solvant organique choisi parmi l'acide acétique ou le dioxanne à une température comprise entre 0 et 50° C, puis, éventuellement, acétyle le produit de formule générale (I) ainsi obtenu au moyen d'anhydride acétique en présence de chlorure de zinc ou en présence d'un mélange de triéthylamine et de diméthylaminopyridine pour obtenir l'acétate du produit de formule (I).

**Claims** for the Contracting States: BE, CH, DE, FR, GB, IT, LI, LU, NL, SE

1. A tocopherol derivative of the general formula (I):

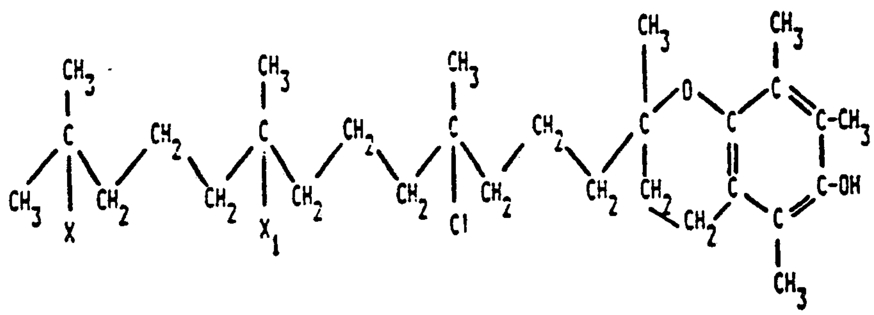

(I)

in which the symbols X and $X_1$, which may be identical or different, represent a hydrogen atom or a chlorine atom, and its acetate.

2. A process for the preparation of a product according to claim 1, characterized in that a product of the general formula (IIa) or (IIb):

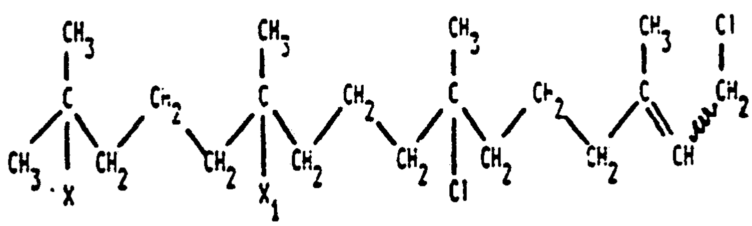

(IIa)

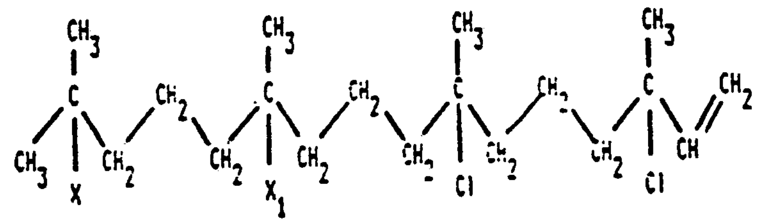

(IIb)

in which the symbols X and $X_1$ are defined as in claim 1, or a mixture of these is condensed with trimethylhydroquinone in an organic solvent chosen from among acetic acid or dioxane at a temperature of between 0 and 50°C and thereafter, where appropriate, the product of the general formula (I), thus obtained, is acetylated with acetic anhydride in the presence of zinc chloride or in the presence of a mixture of triethylamine and dimethylaminopyridine so as to give the acetate of the product of the formula (I).

**Claim** for the Contracting State: AT

A process for the preparation of a tocopherol derivative of the general formula (I):

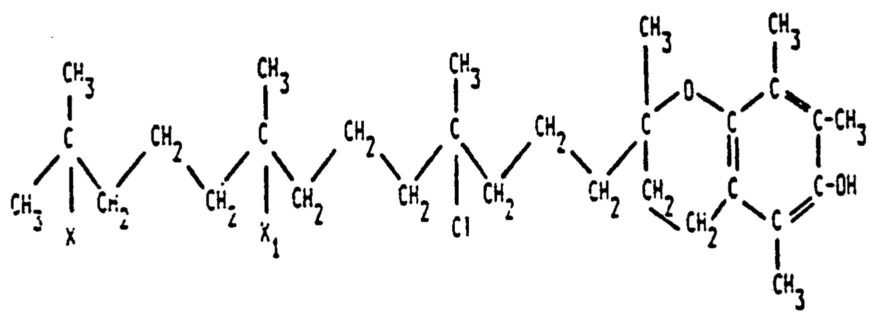

(I)

in which the symbols X and $X_1$, which may be identical or different, represent a hydrogen atom or a chlorine atom, and of its acetate, characterized in that a product of the general formula (IIa) or (IIb):

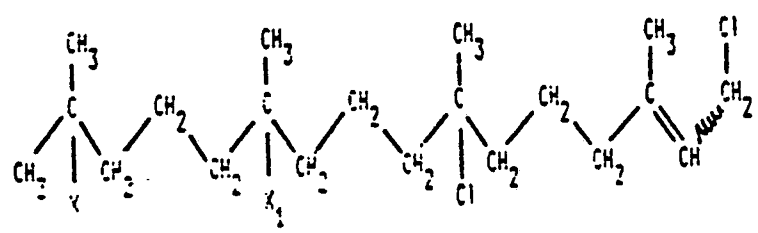

(IIa)

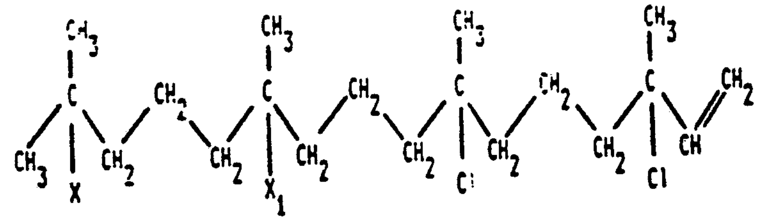

(IIb)

in which the symbols X and $X_1$ are defined as above, or a mixture of these is condensed with trimethylhydroquinone in an organic solvent chosen from among acetic acid or dioxane at a temperature of between 0 and 50°C and thereafter, where appropriate, the product of the general formula (I), thus obtained, is acetylated with acetic anhydride in the presence of zinc chloride or in the presence of a mixture of triethylamine and dimethylaminopyridine so as to give the acetate of the product of the formula (I).

**Patentansprüche** für die Vertragsstaaten: BE, CH, DE, FR, GB, IT, LI, LU, NL, SE

1. Derivat des Tocopherols der allgemeinen Formel (I):

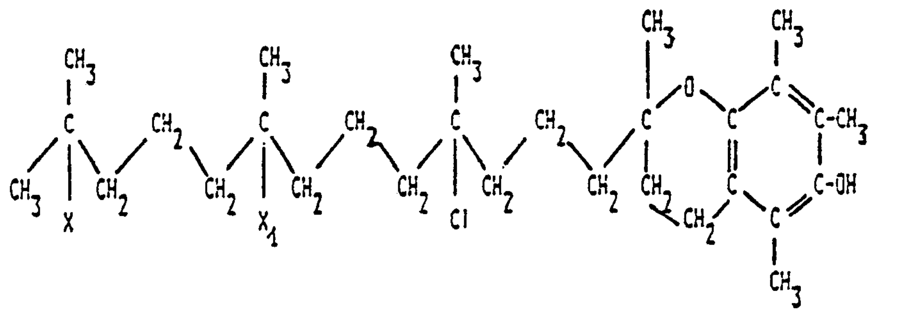

(I)

worin die Symbole X und $X_1$, die identisch oder verschieden sind, ein Wasserstoff- oder Chloratom bedeuten, und sein Acetat.

2. Verfahren zur Herstellung eines Produkts gemäß Anspruch 1, dadurch gekennzeichnet, daß man ein Produkt der allgemeinen Formel (IIa) oder (IIb):

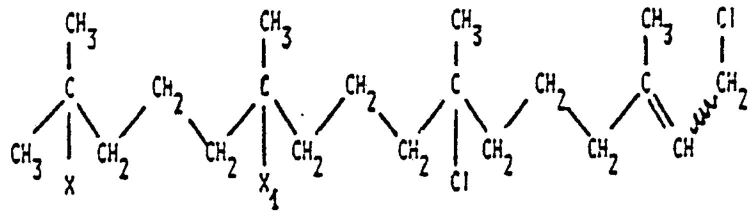

(IIa)

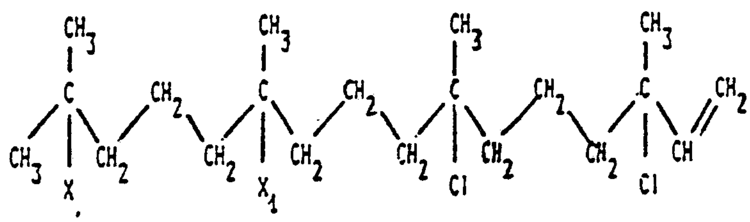

(IIb)

worin die Symbole X und $X_1$ wie in Anspruch 1 definiert sind, oder deren Gemische, mit Trimethylhydrochinon kondensiert, wobei in einem organischen Lösungsmittel, ausgewählt unter Essigsäure oder Dioxan, bei einer Temperatur zwischen 0 und 50°C gearbeitet wird, und daß man dann gegebenenfalls das so erhaltene Produkt der allgemeinen Formel (I) mittels Essigsäureanhydrid in Gegenwart von Zinkchlorid oder in Gegenwart eines Gemisches von Triethylamin und Dimethylaminopyridin acetyliert, um das Acetat des Produkts der Formel (I) zu erhalten.

**Patentanspruch** für den Vertragsstaat: AT

Verfahren zur Herstellung eines Tokopherolderivats der allgemeinen Formel (I):

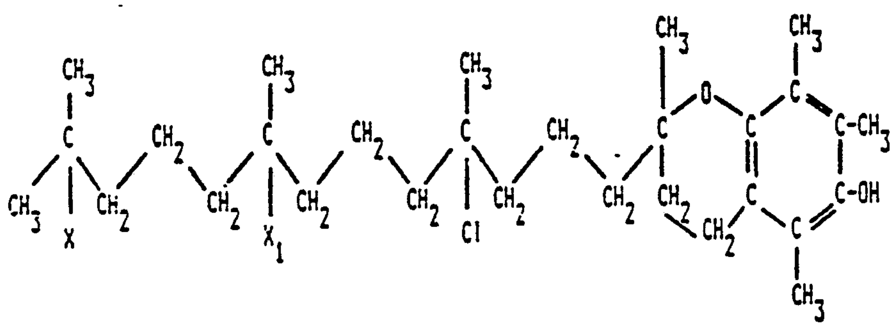
(I)

welcher die Symbole X und $X_1$, die gleich oder voneinander verschieden sind, ein Wasserstoff- oder Chloratom darstellen, und seines Acetats, dadurch gekennzeichnet, daß man eine Verbindung der allgemeinen Formel (IIa) oder (IIb):

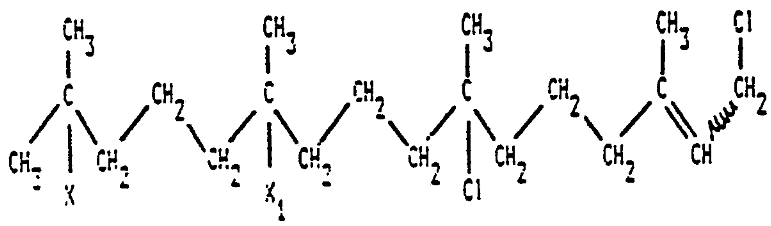
(IIa)

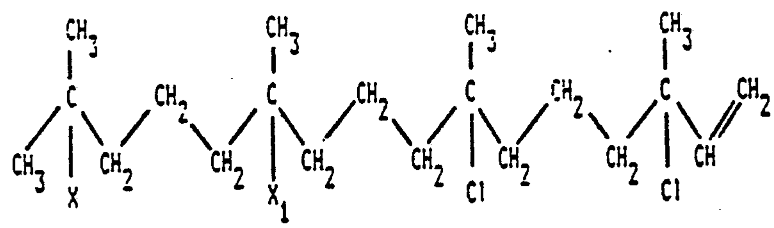
(IIb)

in welcher die Symbole X und $X_1$ die obige Bedeutung haben, oder deren Mischungen mit Trimethylhydrochinon kondensiert, indem man in einem organischen Lösungsmittel, ausgewählt aus Essigsäure oder Dioxan, bei einer Temperatur zwischen 0 und 50°C arbeitet, und dann gegebenenfalls die so erhaltene Verbindung der allgemeinen Formel (I) mit Essigsäureanhydrid in Gegenwart von Zinkchlorid oder in Gegenwart einer Mischung von Triäthylamin und Dimethylaminopyridin unter Bildung des Acetats der Verbindung der Formel (I) acetyliert.